# FASCICULE DE BREVET EUROPEEN

(11) **EP 1 976 454 B1**
(45) Date de publication et mention de la délivrance du brevet: **21.12.2011**
(21) Numéro de dépôt: 07730847.6
(22) Date de dépôt: 23.01.2007
(51) Int. Cl.: A61F 2/06, A61F 2/84

(54) **DISPOSITIF DE TRAITEMENT D'UN CONDUIT DE CIRCULATION DU SANG ET PROCEDE DE PREPARATION ASSOCIE**
VORRICHTUNG ZUR BEHANDLUNG EINER BLUTFÜHRENDEN LEITUNG UND ZUGEHÖRIGES HERSTELLUNGSVERFAHREN
DEVICE FOR TREATING A LINE THROUGH WHICH BLOOD FLOWS AND ASSOCIATED PREPARATION METHOD

(30) Priorité: 24.01.2006 FR 0600654
(43) Date de publication de la demande: 08.10.2008
(73) Titulaire: Laboratoires Perouse, 60173 Ivry Le Temple (FR)
(72) Inventeur: STYRC, Mikolaj, Vitold, L-ECHTERNACH L-6463 (LU); PEROUSE, Eric, F-75016 Paris (FR)
(74) Mandataire: Domenego, Bertrand
(86) Numéro de dépôt international: PCT/FR2007/000129
(87) Numéro de publication internationale: WO 2007/085724

(56) Documents cités:
- EP-A- 0 707 462
- WO-A-02/083038
- US-A- 5 693 083

## Description

La présente invention concerne un dispositif de traitement d'un conduit de circulation du sang selon le preambule de la revendication 1.

Un tel dispositif s'applique au largage dans un vaisseau sanguin d'endoprothèses tubulaires, couramment désignées par le terme anglais « stent » ou d'endovalves tubulaires, comprenant une endoprothèse tubulaire et une valve fixée sur l'endoprothèse,

un dispositif du type précité est décrit dans EP -A- 0 707 462. Une endoprothèse est montée coaxialement sur deux tuteurs creux, aptes à coulisser l'un par rapport à l'autre. Cette endoprothèse est maintenue dans son état rétracté à l'aide de deux liens filiformes la ceinturant à ses extrémités. Les liens filiformes sont engagés respectivement dans des ouvertures de retenue distale et proximale ménagées respectivement dans l'un et l'autre des tuteurs.

Pour le largage de l'endoprothèse, les tuteurs sont déplacés par coulissement relatif de l'un par rapport à l'autre, de sorte que la distance entre les ouvertures de retenue diminue.

La diminution de cette distance provoque le relâchement des liens filiformes et par suite, le déploiement simultané des deux extrémités de l'endoprothèse. L'endoprothèse se déploie ainsi de son état rétracté à son état dilaté sensiblement coaxialement par rapport au tuteur.

Un tel dispositif est donc adéquat dans le cas où le vaisseau sanguin est sensiblement linéaire dans la zone de déploiement de l'endoprothèse.

Toutefois, lorsque l'endoprothèse doit être déployée dans un vaisseau sanguin coudé, par exemple dans une artère raccordée au coeur, la relative rigidité du tuteur ne permet pas de le maintenir sensiblement parallèle à l'axe du vaisseau sanguin au voisinage du point de déploiement de l'endoprothèse.

Le déploiement de l'endoprothèse étant coaxial par rapport à l'axe du tuteur, son positionnement dans la vaisseau sanguin est difficile et fastidieux.

Un but de l'invention est donc de fournir un dispositif de traitement d'un vaisseau sanguin qui peut être positionné de manière plus précise dans un vaisseau sanguin coudé.

A cet effet, l'invention a pour objet un dispositif de traitement selon la revendication 1.

Le dispositif selon l'invention peut comporter une ou plusieurs des caractéristiques des revendications 2 à 8.

L'invention a en outre pour objet un procédé de préparation selon la revendication 9.

L'invention sera mieux comprise à la lecture de la description qui va suivre, donnée uniquement à titre d'exemple, et faite en se référant aux dessins annexés, sur lesquels:
- la Figure 1 est une vue schématique de côté d'un dispositif de traitement selon l'invention, l'endoprothèse étant maintenue dans son état rétracté ;
- la Figure 2 est une vue agrandie, prise en coupe suivant un plan axial médian du dispositif de la Figure 1 ;
- la Figure 3 est une vue analogue à la Figure 1, l'endoprothèse occupant un état dilaté ; et
- la Figure 4 est une vue schématique en coupe partielle du dispositif de la Figure 3 lors de son implantation dans un vaisseau sanguin coudé.

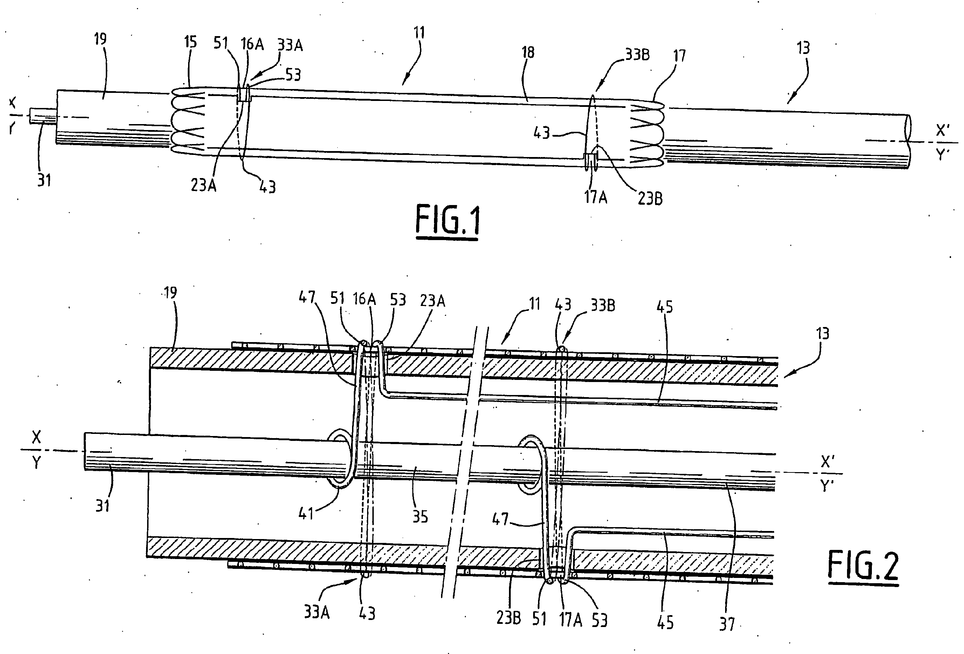

Le dispositif représenté sur les Figures 1 à 4 comporte une endoprothèse tubulaire 11 montée sur un tuteur unique 13 et reliée à ce tuteur 13 par des moyens de retenue libérables.

L'endoprothèse 11 comprend un treillis tubulaire d'axe X-X' en un matériau qui possède des propriétés de ressort. Ainsi, cette endoprothèse est auto-expansible. Le treillis est représenté partiellement les Figures 1 à 4.

Cette endoprothèse est par exemple réalisée par tressage d'un fil unique d'un matériau superélastique, comme décrit dans la demande de brevet européen EP-A-0 857 471.

Le treillis de l'endoprothèse définit, au voisinage d'une extrémité distale 15 de l'endoprothèse 11, un passage 16A de guidage distal des moyens de retenue, et au voisinage d'une extrémité proximale 17 de l'endoprothèse 11, un passage de guidage proximal 17A des moyens de retenue.

Chaque passage de guidage 16A, 17A est délimité par une manille du treillis de l'endoprothèse. Les passages 16A, 17A sont situés respectivement sur deux génératrices de l'endoprothèse 11 décalées angulairement autour de l'axe X-X' de l'endoprothèse 11. Dans cet exemple, les passages 16A, 17A sont situés sensiblement dans un même plan contenant l'axe X-X', de part et d'autre de l'axe X-X'.

En variante, les passages 16A, 17A sont délimités par des anneaux solidaires du treillis et disposés dans le conduit 18.

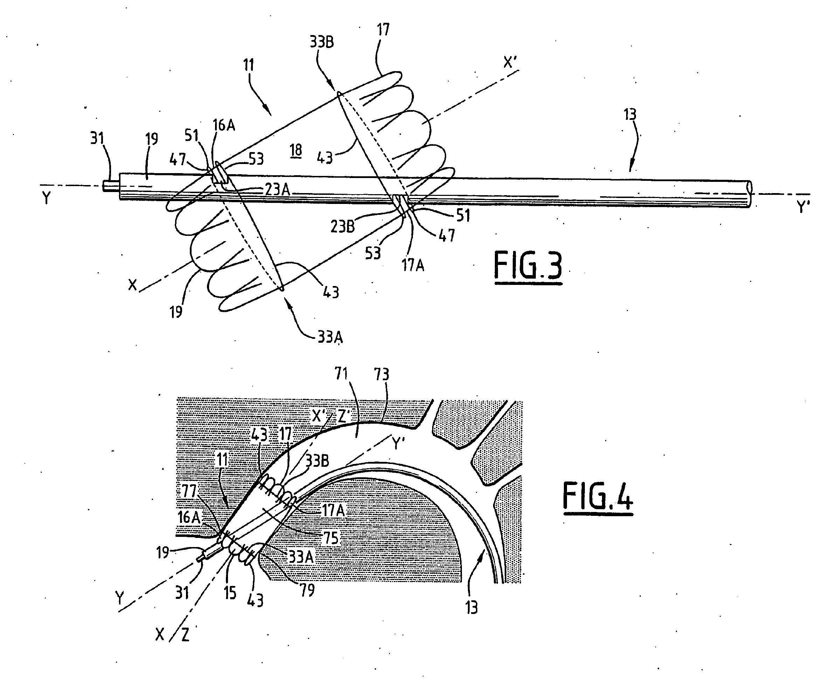

Comme connu en soi, l'endoprothèse 11 est susceptible de se déformer spontanément d'un état comprimé, dans lequel elle présente un petit diamètre (Figure 1) à un état dilaté, dans lequel elle présente un diamètre supérieur (Figure 3), cet état dilaté constituant son état de repos.

Le treillis présente aux extrémités 15, 17 de l'endoprothèse, des fils repliés formant des coudes.

L'endoprothèse 11 délimite intérieurement un conduit 18 de circulation du sang d'axe X-X'.

Dans l'exemple illustré par les Figures 1 et 2, le tuteur 13 comprend un tube flexible creux. Le diamètre interne du tube est adapté pour enfiler celui-ci sur un guide chirurgical filiforme (non représenté) installé sur le patient, préalablement à la mise en place de l'endoprothèse 11 dans un vaisseau sanguin de ce patient.

Le tuteur 13 s'étend longitudinalement entre une extrémité distale 19 destinée à être implantée dans le vaisseau sanguin et une extrémité proximale (non représentée) destinée à être accessible pour un chirurgien.

Des ouvertures de retenue distale et proximale 23A et 23B, décalées longitudinalement, sont ménagées latéralement dans le tuteur 13 au voisinage de l'extrémité distale 19.

Dans cet exemple, les ouvertures 23A et 23B sont ménagées dans un plan longitudinal médian du tuteur 13 de part et d'autre d'un axe Y-Y' du tuteur 13. La distance qui sépare l'ouverture de retenue distale 23A de l'ouverture de retenue proximale 23B est sensiblement égale à la longueur de l'endoprothèse 11 dans son état rétracté, prise suivant l'axe X-X'. L'ouverture de retenue distale 23A s'étend en regard du passage de guidage distal 16A, et l'ouverture de retenue proximale 23B s'étend en regard du passage de guidage proximal 17A.

Comme décrit dans la demande FR-A-2 863 160 de la Demanderesse, le tuteur 13 comprend par ailleurs des embranchements creux (non représentés) au voisinage de son extrémité proximale. Ces embranchements communiquent avec l'intérieur du tuteur 13. Un passage de commande est ménagé à une extrémité libre de chaque embranchement.

Les moyens de retenue libérables de l'endoprothèse 11 comprennent une tige de retenue 31 et des fils de retenue distal et proximal 33A et 33B.

La tige de retenue 31 est disposée dans le tuteur 13. La longueur de la tige 31 est supérieure ou égale à la distance entre l'ouverture de retenue distale 23A et l'extrémité proximale du tuteur 13. Comme illustré sur la Figure 2, elle comprend une partie active 35 et une partie d'actionnement 37.

La tige 31 est mobile en translation le long de l'axe de tuteur Y-Y' dans le tuteur 13, entre une position de retenue dans laquelle la partie active 35 de la tige est située en regard des deux ouvertures de retenue 23A et 23B, une position intermédiaire dans laquelle la partie active 35 est située en regard de l'ouverture de retenue proximale 23B et à l'écart de l'ouverture de retenue distale 23A et une position de libération dans laquelle la partie active 35 est située à l'écart des deux ouvertures de retenue 23A et 23B.

Dans l'exemple représenté sur la Figure 2, le fil de retenue distal 33A comprend un brin unique, qui comporte un passant d'extrémité 41, une boucle de retenue 43, et un tronçon de commande 45.

Le passant d'extrémité 41 est formé à une extrémité distale du brin. Il est constitué d'une boucle fermée de petit diamètre. La partie active 35 de la tige 31 est engagée dans le passant 41, lorsque la tige 31 est dans sa position de retenue.

Le passant 41 est par ailleurs déformable de sorte que sa largeur, lorsqu'il est déformé est sensiblement égale à deux fois la largeur du brin.

Le passant 41 est relié à la boucle de retenue 43 par un tronçon 47, engagé dans l'ouverture de retenue distale 23A et dans le passage de guidage distal 16A.

Comme illustré par la Figure 1, la boucle de retenue 43 est formée par un tronçon du brin, engagé de manière coulissante autour du treillis de l'endoprothèse 11, suivant une circonférence de cette endoprothèse 11, autour de l'axe X-X'. En variante, la boucle de retenue 43 passe successivement à l'intérieur et à l'extérieur du treillis autour de l'axe X-X'.

La boucle de retenue 43 s'étend entre une extrémité de retenue 51 reliée au passant 41 et une extrémité de retenue 53 reliée au tronçon de commande 45. L'extrémité de retenue 53 est engagée dans le passage de guidage 16A et dans l'ouverture de retenue 23A.

La boucle de retenue 43 fixe l'endoprothèse 11 au tuteur 13 au voisinage de l'extrémité distale 19 de ce tuteur 13.

Par ailleurs, la longueur active de la boucle de retenue 43 est variable, de sorte qu'elle contrôle le déploiement de l'endoprothèse 11 par rapport au tuteur 13.

Comme on va le décrire plus bas, la boucle de retenue 43 est extensible entre une configuration de maintien de l'endoprothèse dans son état contracté contre le tuteur 13, dans laquelle elle présente une longueur minimale et un diamètre minimal, et une configuration de libération de l'endoprothèse dans laquelle elle présente une longueur maximale et un diamètre maximal.

Comme représenté sur la Figure 2, le tronçon de commande 45 s'étend dans le tuteur 13 entre l'ouverture de retenue distale 23A et un passage de commande situé à l'extrémité proximale du tuteur 13.

Une extrémité de commande du tronçon de commande 45 est engagée hors du tuteur 13 à travers le passage de commande. Ainsi, une partie de ce tronçon fait saillie hors de l'embranchement. La longueur de cette partie en saillie est variable et commande la longueur de la boucle de retenue.

Ainsi, le déplacement du tronçon de commande 45 par rapport au tuteur 13, vers l'extrémité proximale du tuteur, provoque une diminution correspondante de la longueur active de la boucle de retenue 43, et par suite, le serrage de l'endoprothèse 11 contre le tuteur 13, au niveau de la boucle de retenue 43.

Lorsque l'endoprothèse 11 est dans son état rétracté contre le tuteur 13, le tronçon de commande 45 est maintenu sous tension.

Inversement, le déplacement du tronçon de commande 45 par rapport au tuteur 13 vers l'extrémité distale 19 du tuteur, provoque une augmentation de la longueur active de la boucle de retenue 43 radialement à l'écart de l'axe Y-Y' du tuteur 13 et à l'écart du passage de guidage distal 16A dans un premier sens orienté vers le bas sur la Figure 1. Cette extension de la boucle 43 autorise le déploiement de l'endoprothèse 11 à l'écart du tuteur 13, autour de la boucle de retenue 43.

Lorsque l'endoprothèse 11 est dans son état dilaté, le tronçon de commande 45 est sensiblement détendu.

Comme illustré sur la Figure 2, la structure du fil de retenue proximal 33B est analogue à celle du fil de retenue distal 33A.

Toutefois, à la différence du fil de retenue distal 33A, le déplacement du tronçon de commande 45 du fil de retenue proximal 33B par rapport au tuteur 13, vers l'extrémité distale 19 du tuteur 13 provoque une augmentation de la longueur active de la boucle de retenue 43 radialement à l'écart du tuteur 13 et du passage de guidage proximal 17A dans un deuxième sens opposé au premier sens, orienté vers le haut sur la Figure 1.

Comme illustré par la Figure 3, lorsque l'endoprothèse 11 est dans son état dilaté, et que les boucles de retenue 43 occupent leur configuration détendue de libération de l'endoprothèse 11, l'axe X-X' de l'endoprothèse 11 est incliné par rapport à l'axe Y-Y' du tuteur en l'absence de contrainte extérieure sur l'endoprothèse 11 et/ou sur le tuteur 13.

L'axe X-X' de l'endoprothèse croise l'axe Y-Y' du tuteur dans le conduit intérieur 18, c'est-à-dire qu'en projection dans un plan axial médian passant par l'axe Y-Y' du tuteur, l'axe X-X' de l'endoprothèse coupe l'axe Y-Y' en un point situé à l'intérieur du conduit de circulation 18.

Dans cette configuration, le passage de guidage distal 16A et le passage de guidage proximal 17A sont situés au voisinage du tuteur 13, sensiblement le long de l'axe du tuteur Y-Y'. Cette inclinaison de l'endoprothèse 11 par rapport au tuteur 13 est obtenue spontanément, par exemple en dehors du corps humain, sans qu'il soit nécessaire d'appliquer une contrainte extérieure sur l'endoprothèse 11.

On décrira maintenant comme exemple le fonctionnement du premier dispositif du traitement selon l'invention.

Dans un premier temps, le dispositif est conservé dans un emballage (non représenté), avec l'endoprothèse 11 dans un état déployé analogue à celui représenté sur la Figure 3.

Dans cette configuration, la tige de commande 31 est dans sa position de retenue. Les fils de retenue distal et proximal 23A et 23B sont engagés dans la tige 31 et dans le treillis de l'endoprothèse 11.

Ce conditionnement conserve les propriétés mécaniques de l'endoprothèse 11, particulièrement lorsque le treillis tubulaire de celle-ci est noyé dans un film extensible et étanche, tel qu'un élastomère.

Dans cet état, l'axe X-X' de l'endoprothèse 11 est incliné spontanément par rapport à l'axe Y-Y' du tuteur 13, comme décrit précédemment.

Dans un deuxième temps, le chirurgien extrait le dispositif de son emballage. Il implante un guide chirurgical (non représenté) qui s'étend dans un vaisseau sanguin 71 depuis le point d'introduction extérieur jusqu'à la zone du vaisseau dans laquelle doit être implantée l'endoprothèse tubulaire 11.

Comme illustré par la Figure 4, ce vaisseau 71 présente un coude 73 au voisinage du point d'implantation 75.

Dans un troisième temps, en vue de l'implantation de l'endoprothèse 11 dans le vaisseau sanguin 71, le chirurgien déplace le tronçon de commande 45 de chaque fil de retenue 23A, 23B vers l'extrémité proximale du tuteur 13. La longueur active des boucles de retenue 43 diminue de sorte que l'endoprothèse 11 est rétractée contre le tuteur 13 et fixée solidement par rapport au tuteur 13, coaxialement par rapport au tuteur.

L'endoprothèse 11 est alors dans l'état rétracté illustré sur la figure 1 dans lequel le treillis est sensiblement en appui contre le tuteur 13. Elle est ainsi introduite jusqu'à son lieu d'implantation par déplacement du tuteur 13 le long du guide chirurgical (non représenté).

Dans certains cas, et pour maintenir un encombrement radial minimal, un fourreau (non représenté) est disposé autour de l'endoprothèse 11, avant cette introduction et retiré une fois l'introduction effectuée.

Le tuteur 13 est alors positionné de sorte le passage de guidage proximal 16A est situé au voisinage d'une première surface 77 du vaisseau à traiter, alors que le passage de guidage distal 17A est situé au voisinage d'une surface 79 en regard du vaisseau à traiter. Les passages de guidage 16A et 17A sont placés sensiblement le long de l'axe Y-Y'.

Compte tenu de la relative rigidité du tuteur 13 lorsqu'il est implanté dans le vaisseau 71, le tuteur 13 ne suit pas la forme du vaisseau 71 dans le coude 73. Au voisinage du point d'implantation 75, l'axe Y-Y' du tuteur est incliné par rapport à l'axe Z-Z' du vaisseau 71.

En fonction de la conformation du vaisseau à traiter, le chirurgien peut choisir de déployer en premier l'une ou l'autre des extrémités 15 et 17 de l'endoprothèse 11. On décrira comme exemple le déploiement de l'extrémité distale 15.

Tout d'abord, le chirurgien déplace le tronçon de commande 45 vers l'extrémité distale 19 du tuteur 13. Par suite, la longueur active de la boucle de retenue 43 augmente.

Lors de cette extension de la boucle de retenue 43, le passage de guidage distal 16A est maintenu sensiblement au voisinage de la première surface 77 du vaisseau à traiter, et au voisinage du tuteur 13, alors que la boucle de retenue 43 se déploie à l'écart du tuteur 13 et du passage de guidage distal 16A vers la deuxième surface 79 du vaisseau à traiter.

Le treillis de l'endoprothèse 11 se déforme alors spontanément de l'état comprimé représenté sur la figure 1 à l'état déployé représenté sur la figure 3.

Si le chirurgien n'est pas satisfait du positionnement de l'extrémité distale 15 de l'endoprothèse 11 lorsque celle-ci est déployée, il diminue la longueur active de la boucle de retenue 43 par traction sur le tronçon de commande 45 afin de comprimer l'endoprothèse 11 contre le tuteur 13. L'endoprothèse 11 est alors déplacée jusqu'à une position plus satisfaisante.

De manière analogue, le chirurgien effectue ensuite le déploiement de l'extrémité proximale 17 de l'endoprothèse 11, au moyen du fil de retenue proximal 33B.

La boucle de retenue 43 du fil de retenue proximal 33B se déploie à l'écart du passage de guidage proximal 17A vers la surface opposée 77 du vaisseau à traiter. Les boucles de retenue distale et proximale 43 s'étendent donc, lors de leur déploiement, de part et d'autre de l'axe Y-Y' du tuteur.

Ainsi, lorsque l'endoprothèse 11 occupe sa configuration dilatée dans le vaisseau 71, elle présente un axe X-X' sensiblement parallèle à l'axe Z-Z' du vaisseau 71 au niveau du point d'implantation 75.

Lorsque le chirurgien est satisfait du positionnement de l'extrémité distale 15 de l'endoprothèse 11, il déplace la tige de retenue 31 depuis sa position de retenue jusqu'à la position intermédiaire. Lors de ce déplacement, le passant 41 du fil de retenue distal 33A est libéré de la tige 31.

Le chirurgien tire ensuite sur le tronçon de commande 45 pour amener l'extrémité distale du fil de retenue distal 23A jusqu'au passage de commande, successivement à travers le passage de guidage distal 16A, autour du treillis de l'endoprothèse 11, à l'intérieur du tuteur 13, et à travers l'embranchement de commande.

L'extrémité distale 15 de l'endoprothèse 11 est alors fixée irréversiblement dans le vaisseau sanguin 71.

Le chirurgien vérifie alors le positionnement de l'extrémité proximale 17 de l'endoprothèse 11.

Lorsque cette extrémité 17 est positionnée de manière satisfaisante, il déplace la tige 31 de la position intermédiaire à la position de libération et libère le passant 41 du fil de retenue proximal 33B.

Il retire le fil de retenue proximal 33B comme décrit précédemment pour le fil de retenue distal 33A. L'endoprothèse 11 est disposée en appui sur les parois du vaisseau sanguin 71 et le tuteur 13 est libre par rapport à l'endoprothèse 11. Il est alors retiré hors du vaisseau sanguin.

A cet instant, les moyens de retenue de l'endoprothèse 11 sur le tuteur 13 ont été totalement retirés du vaisseau sanguin.

Grâce à l'inclinaison spontanée de l'axe X-X' de l'endoprothèse 11 par rapport à l'axe Y-Y' du tuteur 13 lorsque les boucles de retenue 43 des liens filiformes 33A, 33B ceinturant l'endoprothèse occupent une configuration de libération de l'endoprothèse 11, le positionnement de l'endoprothèse 11 dans un vaisseau sanguin coudé 71 est facilité. En effet, l'axe X-X' de l'endoprothèse 11 dans son état dilaté est distinct de l'axe Y-Y' du tuteur 13 et est sensiblement confondu avec l'axe Z-Z' du vaisseau sanguin 71 à traiter au voisinage du point d'implantation 75.

En variante, le tuteur 13 peut comprendre deux tronçons télescopiques, mobiles l'un par rapport à l'autre en translation suivant l'axe Y-Y' du tuteur 13, et en rotation autour de l'axe Y-Y' du tuteur 13.

## Revendications

1. Dispositif de traitement d'un conduit (71) de circulation du sang, du type comprenant :
- au moins une endoprothèse tubulaire (11) d'axe (X-X'), déployable radialement entre un état contracté et un état dilaté, l'endoprothèse (11) délimitant un conduit (18) intérieur de circulation du sang ;
- un tuteur (13) d'axe (Y-Y') délimitant au moins une ouverture de retenue proximale (23B) et au moins une ouverture de retenue distale (23A) ;
- un lien filiforme distal (33A) et un lien filiforme proximal (33B), engagés respectivement dans l'ouverture de retendue distale (23A) et dans l'ouverture de retenue proximale (23B), chaque lien (33A, 33B) formant une boucle de retenue (43) ceinturant l'endoprothèse (11), chaque boucle de retenue (43) étant extensible entre une configuration de maintien de l'endoprothèse (11) dans son état contracté contre le tuteur (13), et une configuration de libération de l'endoprothèse (11) dans laquelle l'endoprothèse (11) présente sensiblement son état dilaté,
**caractérisé en ce que**, lorsque chaque boucle de retenue (43) occupe sa configuration de libération, l'axe (X-X') de l'endoprothèse (11) est incliné par rapport à l'axe (Y-Y') du tuteur (13) en l'absence de contrainte extérieure, l'axe (X-X') de l'endoprothèse (11) croisant l'axe (Y-Y') du tuteur (13) dans le conduit intérieur (18).

2. Dispositif selon la revendication 1, **caractérisé en ce que** l'endoprothèse (11) définit un passage de guidage proximal (17A) et un passage de guidage distal (16A), situés respectivement sur deux génératrices de l'endoprothèse (11) décalées angulairement autour de l'axe (X-X') de l'endoprothèse (11), chaque boucle de retenue (43) présentant deux extrémités (51, 53) engagées à travers l'un des passages de guidage (16A, 17A).

3. Dispositif selon la revendication 2, **caractérisé en ce que** le passage de guidage proximal (17A) et le passage de guidage distal (16A) sont situés sensiblement dans un plan axial contenant l'axe (X-X') de l'endoprothèse (11).

4. Dispositif selon l'une des revendications 2 ou 3, **caractérisé en ce que**, lorsque chaque boucle de retenue (43) occupe sa configuration de libération, le passage de guidage proximal (17A) et le passage de guidage distal (16A) sont situés au voisinage du tuteur (13), sensiblement le long de l'axe (Y-Y') du tuteur (13).

5. Dispositif selon l'une quelconque des revendications 2 à 4, **caractérisé en ce que** l'ouverture de retenue distale (23A) est située à l'opposé de l'ouverture de retenue proximale (23B) par rapport à l'axe (Y-Y') du tuteur (13), le passage de guidage distal (16A) étant placé en regard de l'ouverture de retenue distale (23A) et le passage de guidage proximal (17A) étant situé en regard de l'ouverture de retenue proximale (23B).

6. Dispositif selon l'une quelconque des revendications précédentes, **caractérisé en ce qu'**une boucle de retenue proximale (43) se déploie radialement à l'écart du tuteur (13) dans un premier sens, entre la configuration de maintien de l'endoprothèse (11) et la configuration de libération de l'endoprothèse (11), une boucle de retenue distale (43) se déployant radialement à l'écart du tuteur (13) entre sa configuration de maintien de l'endoprothèse (11) et sa configuration de libération de l'endoprothèse (11) dans un deuxième sens opposé au premier sens.

7. Dispositif selon l'une quelconque des revendications précédentes, **caractérisé en ce que** le tuteur (13) présente une extrémité proximale, et une extrémité distale (19) au voisinage de laquelle sont ménagées les ouvertures de retenue (23A, 23B), chaque lien filiforme (33A, 33B) comprenant un tronçon de commande (45) raccordé à une première extrémité (53) de la boucle de retenue (43) et s'étendant jusqu'à l'extrémité proximale du tuteur (13).

8. Dispositif selon la revendication 7, **caractérisé en ce qu'**il comprend une tige (31) de retenue disposée mobile par rapport au tuteur (13) entre une position de retenue dans laquelle une partie active (35) de la tige (31) est placée en regard de chaque ouverture de retenue (23A, 23B) et une position de libération dans laquelle la partie active (35) de la tige (31) est placée à l'écart de chaque ouverture de retenue (23A, 23B), chaque lien filiforme (33A, 33B) comprenant un passant (41) raccordé à une deuxième extrémité (51) de la boucle de retenue (43) et engagé sur la tige (31) dans sa position de retenue.

9. Procédé de préparation d'un dispositif selon l'une quelconque des revendications précédentes, avant son implantation dans un conduit de circulation du sang (71), **caractérisé en ce qu'**il comprend les étapes suivantes :
(a) conservation de l'endoprothèse (11) dans un état dilaté, la ou chaque boucle de retenue (43) étant maintenue dans sa configuration de libération de l'endoprothèse (11), l'axe (X-X') de l'endoprothèse (11) étant incliné par rapport à l'axe (Y-Y') du tuteur (13) en l'absence de contrainte extérieure, l'axe (X-X') de l'endoprothèse (11) croisant l'axe (Y-Y') du tuteur (13) dans le conduit intérieur (18) ; puis
(b) serrage de chaque boucle de retenue (43) pour l'amener dans sa configuration de maintien de l'endoprothèse (11) dans son état contracté contre le tuteur (13), en vue de l'implantation de l'endoprothèse (11) dans le conduit de circulation du sang (71).

## Claims

1. Device for treating a duct (71) through which blood flows, of the type comprising:
at least one tubular endoprosthesis (11) of axis X-X', which can extend radially between a contracted state and a dilated state, the endoprosthesis (11) defining an internal duct (18) through which blood flows;
- a prop (13) of axis Y-Y' defining at least one proximal retention opening (23B) and at least one distal retention opening (23A);
- a wire-like distal tie (33A) and a wire-like proximal tie (33B), engaged respectively in the distal retention opening (23A) and in the proximal retention opening (23B), each tie (33A, 33B) forming a retaining loop (43) encircling the endoprosthesis (11), each retaining loop (43) being extendible between a configuration in which the endoprosthesis (11) is kept in its contracted state against the prop (13), and a configuration in which the endoprosthesis (11) is released and in which the endoprosthesis (11) is substantially in its dilated state, **characterised in that**, when each retaining loop (43) is in its release configuration, the axis (X-X') of the endoprosthesis (11) is inclined with respect to the axis (Y-Y') of the prop (13) when no external stresses are applied, the axis (X-X') of the endoprosthesis (11) intersecting the axis (Y-Y') of the prop (13) in the internal duct (18).

2. Device according to claim 1, **characterised in that** the endoprosthesis (11) defines a proximal guidance passage (17A) and a distal guidance passage (16A), located respectively on two generatrixes of the endoprosthesis (11) which are offset angularly about the axis (X-X') of the endoprosthesis (11), each retaining loop (43) having two ends (51, 53) engaged through one of the guidance passages (16A, 17A).

3. Device according to claim 2, **characterised in that** the proximal guidance passage (17A) and the distal guidance passage (16A) are located substantially in an axial plane containing the axis (X-X') of the endoprosthesis (11).

4. Device according to either claim 2 or claim 3, **characterised in that**, when each retaining loop (43) is in the release configuration thereof, the proximal guidance passage (17A) and the distal guidance passage (16A) are located in the region of the prop (13), substantially along the axis (Y-Y') of the prop (13).

5. Device according to any one of claims 2 to 4, **characterised in that** the distal retention opening (23A) is located opposite the proximal retention opening (23B) with respect to the axis (Y-Y') of the prop (13), the distal guidance passage (16A) being placed in register with the distal retention opening (23A) and the proximal guidance passage (17A) being located in register with the proximal retention opening (23B).

6. Device according to any one of the preceding claim, characterised it, that a proximal retaining, loop (43) extends radially away from the prop (13) in a first direction, between the configuration in which the endoprosthesis (11) is kept in the contracted state and the configuration in which the endoprosthesis (11) is released, a distal retaining loop (43) extending radially away from the prop (13), between the configuration in which the endoprosthesis (11) is kept in the contracted state and the configuration in which the endoprosthesis (11) is released, in a second direction away from the first direction.

7. Device according to any one of the preceding claims, **characterised in that** the prop (93) has a proximal end and a distal end (19) in the region of which the retention openings (23A, 23B) are arranged, each wire-lilce tie (33A. 33B) comprising a control portion (45) connected to a first end (53) of the retaining loop (43) and extending to the proximal end of the prop (13).

8. Device according to claim 7, **characterised in that** it comprises a retention rod (31) which is disposed movably with respect to the prop (13) between a retention position in which an active part (35) of the rod (31) is placed in register with each retention opening (23A, 23B) and a release position in which the active part (35) of the rod (31) is placed apart from each retention opening (23A, 23B), each wire-like tie (33A, 33B) comprising a keeper loop (41) attached to a second end (51) of the retaining loop (43) and engaged on the rod (31) in its retention position.

9. Method of preparation of a device according to any one of the preceding claims, prior to its implantation in a duct through which blood (71) flows, **characterised in that** it comprises the following steps:
(a) keeping the endoprosthesis (11) in a dilated state where the or each retaining loop (43) is kept in its configuration in which the endoprosthesis (11) is released, the axis (X-X') of the endoprosthesis (11) being inclined with respect to the axis (Y-Y') of the prop (13) when no external stresses are applied, the axis (X-X') of the endoprosthesis (11) intersecting the axis (Y-Y') of the prop (13) in the internal duct (18); then
(b) tightening each retaining loop (43) to bring it into the configuration thereof in which the endoprosthesis (11) is kept in its contracted state against the prop (13), in order to implant the endoprosthesis (11) in the duct through which the blood (71) flows.

## Patentansprüche

1. Vorrichtung (71) zur Behandlung einer blutführenden Leitung, umfassend:
- zumindest eine röhrenförmige Endoprothese (11) mit der Achse (X-X'), die radial zwischen einem zusammengezogenen Zustand und einem geweiteten Zustand entfaltbar ist, wobei die Endoprothese (11) eine innere blutführende Leitung (18) definiert;
- eine Stütze (13) mit der Achse (Y-Y'), die zumindest eine proximale Ritckhalteöffnung (23B) und zumindest eine distale Rückhaiteötfnung (23A) definiert;
- ein drahtförmige distales Glied (33A) und ein drahtförmige proximales Glied (33B), die in die distale Rückhalteöffnung (23A) beziehungsweise in die proximale Rückhalteöffnung (23B) eingreifen, wobei jedes Glied (33A, 33B) eine die Endoprothese (11) umgreifend Rückhalteschlinge (43) bilde und jede Rückhalleschhnge (43) zwischen einer Anordnung, bei der die Endoprothese (11) in ihrem zusammengezogener) Zustand gegen die Stütze (13) gehalten wird, und einer Anordnung dehnbar ist, bei der die Endoprothese (11) frei ist und bei der sich die Endoprothese (11) im Wesentlichen in ihrem geweiteten Zustand befindet, **dadurch gekennzeichnet, dass** wenn jede Rüekhafteschlinge (43) ihre freie Anordnung einnimmt, die Achse (X-X') der Endoprothese (11) bezüglich der Achse (Y-Y') der Stütze (13) geneigt ist, wenn keine äußerte Belassung ausgeübt wird, wobei die Achse (X-X') der Endoprothese (11) die Achse (Y-Y') der Stütze (13) in der inneren Leitung (18) schneidet.

2. Vorrichtung gemäß Anspruch 1, **dadurch gekennzeichnet, dass** die Endoprothese (11) einen proximalen Führungskanal (17A) und einen distalen Führungskanal (16A) definiert, die sich jeweils auf zwei Mantellinien der Endoprothese (11) befinden und um die Achse (X-X') der Endoprothese (11) im Winkel versetzt sind, wobei jede Rückhaiteschiinge (43) zwei Enden (51, 53) aufweist, die in einen der Führungskanäle (16A, 17A) eingreifen.

3. Vorrichtung gemäß Anspruch 2, **dadurch gekennzeichnet, dass** der proximale Führungskanal (17A) und der distale Führungskanal (16A) im Wesentlichen in einer die Achse (X-X') der Endoprothese (11) enthaltende Achsenebene liegen.

4. Vorrichtung gemäß einem der Ansprüche 2 oder 3, **dadurch gekennzeichnet, dass** wenn jede Rückhalteschlinge (43) in ihrer freien Anordnung vorliegt, sich der proximal Führungskanal (17A) und der distale Führungskanal (16A) in der Nähe der Stütze (13), im Wesentlichen entlang der Achse (Y-Y') der Stütze (13) befinden.

5. Vorrichtung gemäß einem der Ansprüche 2 bis 4, **dadurch gekennzeichnet, dass** sich die distale Rückhalteöffnung (23A) gegenüber der proximalen Rückhalteöffnung (23B) bezüglich der Achse (Y-Y') der Stütze (13) befindet, wobei der distale Führungskanal (16A) gegenüber der distalen Rückhaiteöffnung (23A) liegt und sich der proximale Führungskanal (17A) gegenüber der proximalen Rückhalteöffnung (23B) befindet.

6. Vorrichtung gemäß einem der vorangehenden Ansprüche, **dadurch gekennzeichnet, dass** sich eine proximale Rückhalteschlinge (43) radial von der Stütze (13) entfernt in einer ersten Richtung zwischen der Anordnung, bei der die Endoprothese (11) gehalten wird, und der Anordnung, bei der die Endoprothese (11) frei ist, erstreckt, wobei sich eine distale Rückhaiteschünge (43) radial von der Stütze (13) entfernt in einer zweiten, der ersten Richtung entgegengesetzten Richtung zwischen der Anordnung, bei der die Endoprothese (11) gehalten wird, und der Anordnung, bei der die Endoprothese (11) frei ist, erstreckt.

7. Vorrichtung gemäß einem der vorangehenden Ansprüche, **dadurch gekennzeichnet, dass** die Stütze (13) ein proximales Ende und ein distales Ende (19) aufweist, in dessen Nähe die Rückhalteöffnungen (23A, 23B) angeordnet sind, wobei jedes drahtförmige Glied (33A, 338) einen Betätigungsabschnitt (45) umfasst, der mit einem ersten Ende (53) der Rückhalteschlinge (43) verbunden ist und sich bis zum proximalen Ende der Stütze (13) erstreckt.

8. Vorrichtung gemäß Anspruch 7, **dadurch gekennzeichnet, dass** sie einen Haltestab (31) umfasst, der bezüglich der Stütze (13) zwischen einer Rückhaltestellung, bei der sich ein aktiver Teil (35) des Stabs (31) gegenüber jeder Rückhaiteöffinung (23A, 23B) befindet, und einer freien Stellung, bei der sich der aktive Teil (35) des Stabs (31) von jeder Rückhalteötinung (23A, 23B) entfernt befindet, beweglich angeordnet ist, wobei jedes drahtförmige Glied (33A, 33B) eine Schlaufe (41) umfasst, die an einem zweiten Ende (51) der Ruckhatteschiinge (43) befestigt ist und auf dem Stab (31) in seiner Rückhaltestellung eingreift.

9. Verfahren zur Herstellung einer Vorrichtung gemäß einem der vorangehenden Ansprüche vor seiner Implantation in eine blutführende Leitung (71), **dadurch gekennzeichnet, dass** es die folgenden Schritte umfasst:
(in) Halten der Endoprothese (11) in einem geweiteten Zustand, wobei die oder jede Rückhalteschlinge (43) in ihrer Anordnung gehalten wird, bei der die Endoprothese {11} frei ist, die Achse (X-X') der Endoprothese (11) in Abwesenheit von äußerer Belastung bezüglich der Achse (Y-Y') der Stütze (13) geneigt ist und die Achse (X-X') der Endoprothese (11) die Achse (Y-Y') der Stütze (13) in der inneren Leitung (18) schneidet, und dann
(b) Spannen jeder Rückhalteschlinge (43), um sie in ihre Anordnung zu bringen, bei der die Endoprothese (11) zur Implantation der Endoprothese (11) in die blutführende Leitung (71) in ihrem zusammengezogenen Zustand gegenüber der Stütze (13) gehalten wird.
